# EUROPEAN PATENT SPECIFICATION

(11) **EP 1 930 326 B1**
(45) Date of publication and mention of the grant of the patent: **13.05.2009**
(21) Application number: 06025009.9
(22) Date of filing: 04.12.2006
(51) Int. Cl.: C07D 265/16, C08G 8/28, C08G 14/06, C08G 59/30, C08G 59/50

(54) **Synthesis of new dihydrobenzoxazine**
Synthese von neuem Dihydrobenzoxazin
Synthèse d'une nouvelle dihydrobenzoxazine

(43) Date of publication of application: 11.06.2008
(73) Proprietor: NAN YA PLASTICS CORPORATION, Taipei (TW)
(72) Inventor: Tzou, Ming-Jen, Taipei (TW)
(74) Representative: Viering, Jentschura & Partner

(56) References cited:
- EP-A2- 0 789 056
- WO-A-20/04101509
- JP-A- 2000 007 901
- US-A1- 2002 128 354
- ESPINOSA M A ET AL: "Novel phosphorilated flame retardant thermosets: epoxy-benzoxazine-novolac systems" POLYMER, ELSEVIER SCIENCE PUBLISHERS B.V, GB, vol. 45, no. 18, 19 August 2004 (2004-08-19), pages 6103-6109, XP004549790 ISSN: 0032-3861

## Description

### FIELD OF THE INVENTION

The present invention relates to an epoxy resin varnish composition with high glass transition temperature for laminate plate, which is of good reactivity and wide working window in the manufacturing process. It shows good working ability, and the substrate made therefrom is of high glass transition temperature (Tg), low water absorption and excellent heat resistance, and are qualified for UL94 V-0 Flammability Test. Fire retardant requirement is fulfilled by effectively reducing usage of phosphorus or bromine and the cost is decreased thereby.

### DESCRIPTION OF THE RELATED PRIOR ART

Epoxy resin is of good reactivity, toughness and softness due to its chemical structure, as well as good mechanical and electric properties and dimension stability; it also shows excellent adherence on various substrates. Cured epoxy resin retains its original characteristics, especially for blocking vapor and chemical substances, and has the advantages of lightweight and low-cost. Therefore, epoxy resin is widely used in the fields of electronics and aerospace, particularly used as semiconductor packaging materials and printed circuit board substrates. As the development of printed circuit boards is toward light, thin and more precious in recent years, there is still a need for improving qualities of these materials.

### SUMMARY OF THE INVENTION

It is necessary for laminate plate usable in printed circuit to be lead-free according to the international regulation due to the trend of environmental protection, so that the working ability of the obtained substrate is strictly regulated. Particularly, issues involving glass transition temperature (Tg) of materials and heat resistance of substrate in solder furnace are required to be solved by those who are in this field.

Accordingly, the present inventor has conducted extensively study and found that the above required properties can be obtained by epoxy resin varnish admixing a new dihydrobenzoxazine (NPBX) thermosetting resin with proper reactivity, wide working window, high glass transition temperature, excellent heat resistance, low water absorption and good electronic properties. Dihydrobenzoxazine thermosetting resin is of good heat resistance, high Tg and fire retardance by containing benzene ring and nitrogen in its chemical structure. Further, with steric hindrance by adequate number of epoxy functional groups and benzene rings. The proper reactivity and higher resin molten viscosity are obtained, thus working window is wide and the operations are excellent. Also, as NPBX thermosetting resin is a nitrogen-containing heterocyclic compound with minor amount of phosphorus introduced thereto, better resinous materials for fire retardant laminate plate are obtained from synergistic effect of combining phosphorus and nitrogen.

Therefore, an object of the present invention is to provide an epoxy resin varnish composition with high glass transition temperature for laminate plate, wherein the resin composition comprises: (A) a new dihydrobenzoxazine (NPBX) thermosetting resin obtained by reacting the following compounds: (a) phenolic products from the reaction of di- or multifunctional epoxy resin and di-functional phenolic compound; (b) mono- or di-functional primary amines; (c) di-functional phenols; and (d) formaldehyde or paraformaldehyde, (B) one or more epoxy resins, (C) novolac resin curing agents, and (D) curing promoters.

With the epoxy resin varnish composition, after cured, glass transition temperature (Tg) of the resultant materials is effectively elevated, water absorption of the obtained substrate is lowered, and heat resistance is improved. Therefore, it is cost-effective while having excellent working ability.

By admixing a new NPBX thermosetting resin, the epoxy resin varnish composition with high glass transition temperature for laminate plate according to the present invention is of better mechanical, chemical and physical properties like low hygroscopicity, high glass transition temperature, good dimension stability and electric properties, as compared with the general epoxy resin. It is ring-open polymerized by itself at higher temperature (230°C) without adding promoter, and no volatile substance (by product) is produced during the ring-open polymerization so that the obtained substrate is not affected physically. However, curing reaction rate at high temperature is too fast to control. Therefore, if adequate phenols are added during curing, curing speed is moderated, curing temperature is lowered, and ring-open polymerization is promoted to completion, so that the obtained substrate is of better physical properties.

The NPBX thermosetting resin according to the present invention is described as prepared with aniline, bisphenol A and formaldehyde in US Patent No. 4,607,091, while utilizing novolac resin, aniline and formaldehyde in US Patent No. 6,005,064. However, the NPBX thermosetting resins made by the above patents are of poor solubility and is even saturated during storage at room temperature to cause that a large amount of crystals are precipitated out, so that it is inconvenient for subsequent use. To prevent from precipitating out, the present invention uses modified and synthesized NPBX resin, as its structure has been changed so that no large-amount precipitation occurs during storage, and which is useful for mass production industrially. Also, it is descried in US Patent No. 5,021,484 to achieve UL94 V-0 grade by directly curing at high temperature and adding Al(OH)₃ fire retardance; and US Patent No. 5,443,911 is to utilize benzoxazine, brominated epoxy resin and curing agent for reducing the usage of bromine, and after curing the resultant laminate plate achieves fire retardant grade. Additionally, it is described in ROC Patent No. 091125399 to utilize aromatic diamines, phenols, aniline, and aldehydes for producing NPBX thermosetting resin; however, it is expensive to use aromatic diamines and is not suitable for industrial uses.

### DETAILED DESCRIPTION OF THE INVENTION

The present invention relates to an epoxy resin varnish composition comprising: (A) a new NPBX thermosetting resin, (B) one or more epoxy resins, (C) novolac resin curing agents, and (D) curing promoters. An NPBX thermosetting resin containing varnish composition for laminate plate is obtained by mixing (A), (B), (C), and (D) at specific ratio.

The component (A) with the following structure of a new NPBX thermosetting resin is obtained by reacting (a) phenolic products obtained from the reaction of di- or multifunctional epoxy resin and di-functional phenolic compounds; (b) mono- or di-functional primary amines; (c) di-functional phenols; and (d) formaldehyde or paraformaldehyde, in the presence of hydrocarbon solvents. Various types are provided through the modification as fully described in the following synthesis examples: 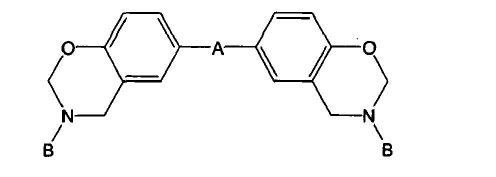 wherein A represents the following formula: 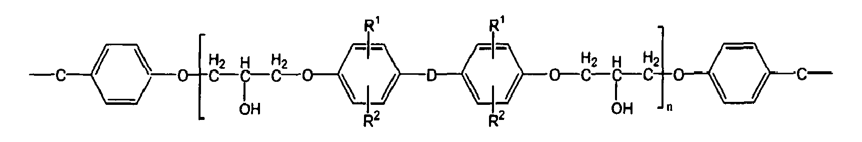 C represents 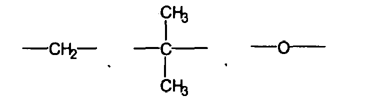 D represents

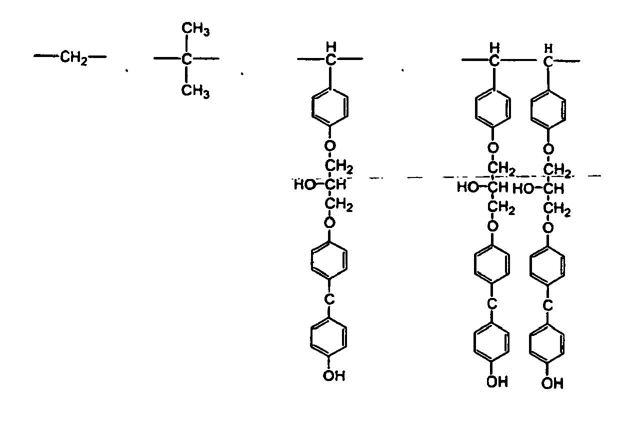

R¹ and R² each independently represents H, CH₃ or Br;
and B represents amines or diamines.
(A-1) NPBX-610 (80 solid% of modified NPBX) is obtained by charging 1710 g of bisphenol A (BPA), 558 g of liquid epoxy resin of NPEL-128E and quaternary phosphor salt catalyst into a 5L 4-neck glass reactor fitted with heating jacket, temperature controller, electric stirrer and condenser, in the presence of small amount of solvents, and reacting at 150°C for 2 hours; after cooling, adding 782 g of paraformaldehyde and 1000 g of toluene to dissolve under stirring, and gradually dropping 1116 g of aniline into the above solution; reacting while keeping temperature at 60-110°C for 4~6 hours, distilling solvents and non-reacted reactants under vacuum, and adding 900 g of acetone to dissolve them.
(A-2) NPBX-620 (80 solid% of modified NPBX) is obtained by charging 1710 g of BPA, 279 g of NPEL-128E and quaternary phosphor salt catalyst into a 5L 4-neck glass reactor fitted with heating jacket, temperature controller, electric stirrer and condenser, in the presence of small amount of solvents, and reacting at 150°C for 2 hours; after cooling, adding 880 g of paraformaldehyde, 148 g of diaminodiphenylmethane and 1000 g of toluene to dissolve under stirring, and gradually dropping 1116 g of aniline into the above solution; reacting while keeping temperature at 60-110°C for 4~6 hours, distilling solvents and non-reacted reactants under vacuum, and adding 900 g of acetone to dissolve them.
(A-3) NPBX-630 (80 solid% of modified NPBX) is obtained by charging 1710 g of BPA, 600 g of tetrafunctional epoxy resin NPPN-431 and quaternary phosphor salt catalyst into a 5L 4-neck glass reactor fitted with heating jacket, temperature controller, electric stirrer and condenser, in the presence of small amount of solvents, and reacting at 150°C for 2 hours; after cooling, adding 782 g of paraformaldehyde and 1000 g of toluene to dissolve under stirring, and gradually dropping 1116 g of aniline into the above solution; reacting while keeping temperature at 60-110°C for 4~6 hours, distilling solvents and non-reacted reactants under vacuum, and adding 900 g of acetone to dissolve them.
(A-4) NPBX-640 (80 solid% of modified NPBX) is obtained by charging 1710 g of BPA, 300 g of tetrafunctional epoxy resin NPPN-431 and quaternary phosphor salt catalyst into a 5L 4-neck glass reactor fitted with heating jacket, temperature controller, electric stirrer and condenser, in the presence of small amount of solvents, and reacting at 150°C for 2 hours; after cooling, adding 880 g of paraformaldehyde, 148g of diaminodiphenylmethane and 1000 g of toluene to dissolve under stirring, and gradually dropping 1116 g of aniline into the above solution; reacting while keeping temperature at 60-110°C for 4~6 hours, distilling solvents and non-reacted reactants under vacuum, and adding 900 g of acetone to dissolve them.

In the synthesis examples A-1 to A-4, component (a) phenolic products are obtained by the reaction of di- or multifunctional epoxy resin and di-functional phenolic compounds for preparing NPBX. It is generally the phenolic products with the following formula from the reaction of liquid epoxy resin and BPA: 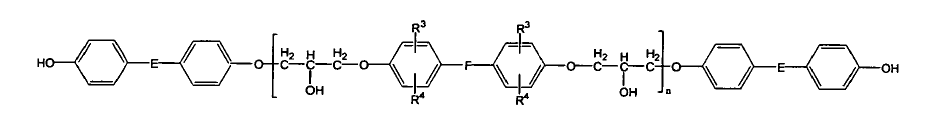 wherein F represents

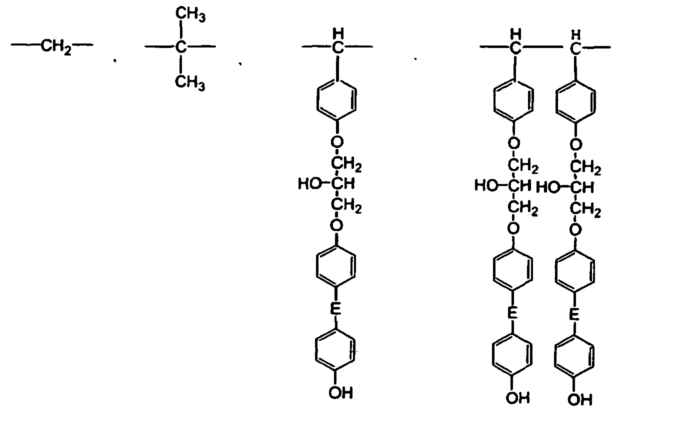

E represents

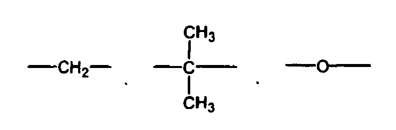

and R³ and R⁴ each independently represents H, CH₃ or Br.

Particularly, the present invention pertains to an epoxy resin varnish composition with high glass transition temperature for laminate plate, which is admixed with dihydrobenzoxazine thermosetting resin, comprising: (A) 20-70 wt% of dihydrobenzoxazine thermosetting resin, (B) 30-70 wt% of one or more epoxy resins , (C) 1-20 wt% of novolac resin curing agent, and (D) curing promoter, with usage being 0.1 PHR based on total amount of resin,
wherein the dihydrobenzoxazine thermosetting resin has the following formula: 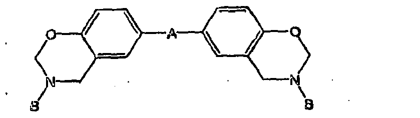 wherein A represents the following formula: 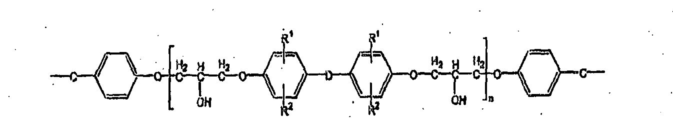 C represents 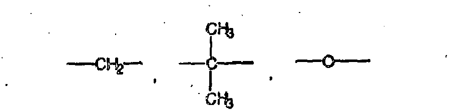 D represents 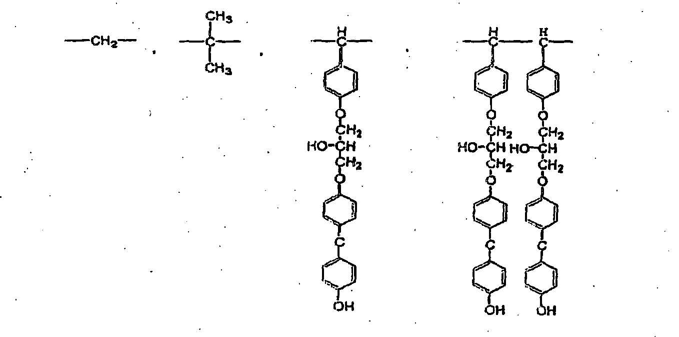 R¹ and R² each independently represents H, CH₃ or Br;
and B represents amines or diamines.

Component (b) mono-or-di-functional primary amines for preparing NPBX is generally primary amines such as methyl amine, aniline, toluidine, anisidine, etc., or aliphatic or aromatic amines, preferably aniline or diaminodiphenylmethane.

Component (c) di-functional phenols for preparing NPBX is generally o-cresol, bisphenol A, bisphenol F, bisphenol S, melamine, or novolac resin, preferably bisphenol or novolac resin.

Component (d) formaldehyde or paraformaldehyde for preparing NPBX is generally formaldehyde, paraformaldehyde or formaldehyde vapor, preferably formaldehyde as it dissolves slowly with addition of primary amine to promote the formation of NPBX.

Solvent for preparing NPBX is not particularly limited, as long as it is capable to dissolve various reactants. For example, it is selected from alcohols, ketones, ethers, esters, or hydrocarbons, preferably hydrocarbons such as toluene and xylene, since water is generated after polymerization, which is immiscible with toluene so that is able to reuse after reflux distillation.

As to epoxy resin in component (B) of the resin varnish composition according to the present invention, it can be phosphine-containing epoxy resin obtained by reacting organic phosphine compounds (for example, 9,10-dihydro-9-oxa-10-phosphorphenanthrene-10-oxo compound (DOPO)) and o-cresol formaldehyde novolac epoxy (NPCN-703), with phosphorus content being 2~5 wt%; or brominated epoxy resin obtained by reacting tetrabromobisphenol A (TBBA) and liquid epoxy resin (NPEL-128E) and admixing multifunctional epoxy resin, with bromine content being 14~21 wt%.

As to curing agent in component (C) of the resin varnish composition according to the present invention, it comprises novolac resin curing agents such as polyvalent amines, polyvalent carboxylic acids, dicyano diamides, anhydrides, phenol novolac epoxy (PN), melamine phenol novolac (MPN), BPA phenol novolac (BPA-PN), and tetraphenol ethane resin (TPN, manufactured by Nan Ya Plastics Corporation), preferably tetraphenol ethane resin.

As to curing promoter in component (D) of the resin varnish composition according to the present invention, it comprises terniary phosphine, terniary amine, quaternary phosphonium, quaternary ammonium, and imidazole, wherein terniary phosphine comprises triphenylphosphine, and the like; terniary amine comprises trimethyl aniline, triethyl amine, tributyl amine, dimethylamino ethanol, and the like; quaternary phosphonium comprises halo-containing quaternary phosphonium such as tetrabutyl phosphonium bromide, tetraphenyl phosphonium bromide, ethyl triphenyl phosphonium bromide, propyl triphenyl phosphonium bromide, butyl triphenyl phosphonium bromide, and the like; quaternary ammonium comprises halo-containing quaternary ammonium such as tetramethyl ammonium bromide, tetraethyl ammonium bromide, tetrabutyl ammonium bromide, triethyl benzyl ammonium bromide, triethyl phenethyl ammonium bromide, and the like; imidazole comprises 2-methyl imidazole, 2-ethyl imidazole, 2-dodecanyl imidazole, 2-phenyl imidazole, 4-methyl imidazole, 4-ethyl imidazole, 4-dodecanyl imidazole, 2-ethyl-4-methyl imidazole, 2-ethyl-4-hydroxymethyl imidazole, and the like, preferably 2-methyl imidazole or 2-ethyl-4-methyl imidazole. The promoter can be used singly or as mixture of two or more thereof. The usage is 0.01~1 PHR, preferably 0.04~0.15 PHR based on total amount of resin.

An epoxy resin varnish composition with high glass transition temperature for laminate plate, according to the present invention, comprises: (A) 20~70 wt% of NPBX-610, 620, 630 or 640, (B) 30~70 wt% of phosphorus-containing epoxy resin (NPEP, with phosphorus content being 2~5 wt%) or bromine-containing epoxy resin (NPEB, with bromine content being 14~21 wt%), (C) 1~20 wt% of curing agent, and (D) curing promoter, with usage being 0.01~1 PHR based on total amount of resin. It is possible to add inorganic fillers (silica, aluminum hydroxide, etc.) or other modifiers (plasticizer, light stabilizer, thermal stabilizer, UV absorber, etc.), and add solvents for modifying viscosity of varnish such as organic aromatic solvents, protic solvents, keto solvents, ether solvents, and ester solvents, suitably toluene, N,N-dimethylformamide, acetone, methyl ethyl ketone, 1-methoxy-2-propanol, ethyl acetate, etc. As to manufacture laminate plate, a sheet of fiber glass cloth is impregnated with the composition, and then is heated and dried to be prepreg; the prepreg is covered with copper foil on one or both sides, and some are laminated and heated under pressure to obtain a copper foil substrate.

Curing temperature for the epoxy resin varnish composition according to the present invention can be 30 to 300°C, preferably 150 to 210°C. If curing temperature is too low, curing rate is too slow that curing time has to be prolonged and it is ineffective for production; on the other hand, if curing temperature is too high, resin tends to be degraded, and curing rate is too fast to control. With the addition of NPBX to the composition, the cost is reduced; with the addition of phenolic curing agent to the composition, the reaction temperature is lowered and curing is promoted to completion, so that substrate Tg and other properties are improved.

### EXAMPLE

The explanation is descried in detail in the present invention by the following preferred embodiments. Designations and ingredients thereof used in the Examples and Comparative Examples are listed as below.
Epoxy resin A: Phosphorus -containing epoxy resin manufactured by Nan Ya Plastics Corporation; trade name: NPEP-200LA70, epoxy equivalent: 340~380 g/eq, phosphorus content: 2.6%.
Epoxy resin B: Phosphorus -containing epoxy resin manufactured by Nan Ya Plastics Corporation; trade name: NLEP-204A70, epoxy equivalent: 440~480 g/eq, phosphorus content: 4%.
Epoxy resin C: Bromine-containing epoxy resin manufactured by Nan Ya Plastics Corporation; trade name: NPEB-454A80, epoxy equivalent: 420~455 g/eq, bromine content: 19%.
Epoxy resin D: Bromine-containing epoxy resin manufactured by Nan Ya Plastics Corporation; trade name: NPEB-485A80, epoxy equivalent: 385~405 g/eq, bromine content: 19%.
Curing agent A: Dicyanamide (DICY), 14.7% in DMF.
Curing agent B: Novolac resin manufactured by Nan Ya Plastics
Corporation; trade name: BPA-PN.
Curing agent C: Tetraphenol ethane resin manufactured by Nan Ya Plastics Corporation; trade name: TPN.
Promoter 2MI: 2-Methyl imidazole, 14.2% in DMF.

### Examples 1-8

Phosphorus-containing epoxy resin, NPBX and curing agent were used at various ratio, and inorganic fillers (Al(OH)₃ and SiO₂) and curing promoter were added, as described in Table 1; then varnish resin compositions at 65 solid% were obtained by adjusting with acetone. Laminate plates were prepared by conventional process, wherein a sheet of 7628 fiber glass cloth was impregnated with the above varnish resin solution and was dried at 170°C (impregnator temperature) for several minutes. Molten viscosity of dried prepreg was adjusted to 4000~10000 poises by controlling drying time, and 8 films made therefrom were laminated between 2 sheets of copper foils each with thickness of 35µm under the pressure of 25kg/cm². Temperature raising was controlled as below. Thus, a laminate plate was obtained after hot press.

| | | | | | | | | |
|---|---|---|---|---|---|---|---|---|
| 85°C | → | 85°C | → | 200°C | → | 200°C | → | 130°C |
| 20 min | | 30 min | | 120 min | | cooled slowly | | |

The obtained copper foil substrates were 1.6 mm in thickness.

### Comparative Examples 1-2

Phosphorus-containing epoxy resin and curing agent but no NPBX were used at various ratio, and inorganic fillers (Al(OH)₃ and SiO₂) and curing promoter were added; then varnish resin compositions at 65 solid% were obtained by adjusting with acetone. Laminate plates were prepared in the same manner as Examples 1-8.

### Examples 9-16

Brominated epoxy resin, NPBX and curing agent were used at various ratio, and curing promoter was added, as described in Table 2; then varnish resin compositions at 65 solid% were obtained by adjusting with acetone. Laminate plates were prepared in the same manner as Examples 1-8.

### Comparative Examples 3-4

Brominated epoxy resin and curing agent but no NPBX were used at various ratio, and curing promoter was added; then varnish resin compositions at 65 solid% were obtained by adjusting with acetone. Laminate plates were prepared in the same manner as Examples 1-8.

### Measurements

### 1. Varnish Gel Time (VGT)

Varnish solutions were made by mixing epoxy resin solution and various curing agents and promoters, then 0.3 ml of varnish solution was dropped on heat plate at 170°C to measure gel time.

### 2. Water Absorbtion

Etched substrates were cut into square sheet of 5 cm² and baked in oven at 105°C for 2 hours. Sample was put into pressure cooker under condition of 2 atm × 120°C for 30 minutes. Weight difference of the sample before and after pressure cooker processing was divided by initial weight to calculate water absorbtion.

### 3. 288°C Solder Heat Resistance

Sample processed in pressure cooker as above was immersed into solder furnace at 288°C to measure delamination time due to sample explosion.

**Table 1: Compositional Percentage of Phosphor-containing Epoxy Resin Varnishes Made by Stirring at Normal (Room) Temperature**

| | Example 1 | Example 2 | Example 3 | Example 4 | Example 5 | Example 6 | Example 7 | Example 8 | Comparative Example 1 | Comparative Example 2 |
|---|---|---|---|---|---|---|---|---|---|---|
| Epoxy resin A | **58** | **54** | **57** | **58** | | | | | **97** | |
| Epoxy resin B | | | | | **41** | **39** | **37** | **38** | | **79** |
| NPBX-610 | **26** | | | | **47** | | | | | |
| NPBX-620 | | **30** | | | | **47** | | | | |
| NPBX-630 | | | **26** | | | | **50** | | | |
| NPBX-640 | | | | **28** | | | | **45** | | |
| Curing agent A | | | | | | | | | **3** | |
| Curing agent B | **16** | | **17** | | | | **13** | | | **21** |
| Curing agent C | | **16** | | **14** | **12** | **14** | | **17** | | |
| Filler SiO2/ATH | **30** | **30** | **30** | **30** | **30** | **30** | **30** | **30** | **30** | **30** |
| Promoter 2MI(PHR) | **0.11** | **0.08** | **0.06** | **0.1** | **0.1** | **0.08** | **0.07** | **0.07** | **0.05** | **0.08** |

**Table 2: Compositional Percentage of Bromine-containing Epoxy Resin Varnishes Made by Stirring at Normal (Room) Temperature**

| | Example 9 | Example 10 | Example 11 | Example 12 | Example 13 | Example 14 | Example 15 | Example 16 | Comparative Example 3 | Comparative Example 4 |
|---|---|---|---|---|---|---|---|---|---|---|
| Epoxy resin C | **53** | **50** | **49** | **50** | | | | | **97** | |
| Epoxy resin D | | | | | **46** | **44** | **45** | **47** | | **78** |
| NPBX-610 | **31** | | | | **38** | | | | | |
| NPBX-620 | | **33** | | | | **44** | | | | |
| NPBX-630 | | | **37** | | | | **42** | | | |
| NPBX-640 | | | | **35** | | | | **39** | | |
| Curing agent A | | | | | | | | | **3** | |
| Curing agent B | **16** | | **14** | | | **12** | | | | **22** |
| Curing agent C | | **17** | | **15** | **16** | | **13** | **14** | | |
| Promoter 2MI(PHR) | **0.06** | **0.07** | **0.05** | **0.06** | **0.09** | **0.05** | **0.04** | **0.06** | **0.08** | **0.1** |

**Table 3: Evaluation on Substrates Made of Various Compositions**

| Item | Example 1 | Example 2 | Example 3 | Example 4 | Example 5 | Example 6 | Example 7 | Example 8 | Comparative Example 1 | Comparative Example 2 |
|---|---|---|---|---|---|---|---|---|---|---|
| Varnish gel time, sec^{*1} | **320** | **330** | **350** | **300** | **380** | **290** | **330** | **340** | **310** | **340** |
| Glass transition temp, °C^{*2} | **170** | **182** | **173** | **186** | **192** | **189** | **182** | **195** | **150** | **153** |
| Water absorption, %*3 | **0.21** | **0.19** | **0.15** | **0.20** | **0.21** | **0.18** | **0.17** | **0.16** | **0.30** | **0.28** |
| Solder heat resistance, sec*⁴ | **400** | **600** | **450** | **500** | **400** | **460** | **480** | **590** | **380** | **390** |
| Fire retardance (UL-94) | **V-0** | **V-0** | **V-0** | **V-0** | **V-0** | **V-0** | **V-0** | **V-0** | **V-0** | **V-0** |
| Note: | | | | | | | | | | |
| *1. 0.3 ml of varnish resin was dropped on heat plate at 170°C to measure gel time. | | | | | | | | | | |
| *2. Differential scanning calorimeter (DSC) was used with temperature raising rate = 20°C/min. | | | | | | | | | | |
| *3. Sample was heated at 120°C under 2 atm in pressure cooker for 30 minutes. | | | | | | | | | | |
| *4. Sample was heated at 120°C under 2 atm in pressure cooker for 30 minutes, and immersed into solder fumace at 288°C to measure delamination time due to sample explosion. | | | | | | | | | | |

**Table 4: Evaluation on Substrates Made of Various Compositions**

| Item | Example 9 | Example 10 | Example 11 | Example 12 | Example 13 | Example 14 | Example 15 | Example 16 | Comparative Example 3 | Comparative Example 4 |
|---|---|---|---|---|---|---|---|---|---|---|
| Varnish gel time, sec^{*1} | **310** | **320** | **340** | **295** | **320** | **295** | **310** | **330** | **320** | **310** |
| Glass transition temp, °C^{*2} | **165** | **173** | **168** | **176** | **177** | **182** | **180** | **179** | **149** | **151** |
| Water absorption. %^{*3} | **0.20** | **0.21** | **0.19** | **0.22** | **0.16** | **0.17** | **0.19** | **0.19** | **0.28** | **0.30** |
| Solder heat resistance, sec ^{*4} | **600** | **560** | **500** | **600** | **500** | **600** | **520** | **590** | **400** | **500** |
| Fire retardance (UL-94) | **V-0** | **V-0** | **V-0** | **V-0** | **V-0** | **V-0** | **V-0** | **V-0** | **V-0** | **V-0** |
| Note: | | | | | | | | | | |
| *1. 0.3 ml of varnish resin was dropped on heat plate at 170°C to measure gel time. | | | | | | | | | | |
| *2. Differential scanning calorimeter (DSC) was used with temperature raising rate = 20°C/min. | | | | | | | | | | |
| *3. Sample was heated at 120°C under 2 atm in pressure cooker for 30 minutes. | | | | | | | | | | |
| *4. Sample was heated at 120°C under 2 atm in pressure cooker for 30 minutes, and immersed into solder fumace at 288°C to measure delamination time due to sample explosion. | | | | | | | | | | |

The result of substrate test is shown in Table 3. The halo-free resin compositions in the present invention are qualified for UL94 V-0 Flammability Test and improve other properties of substrate with phosphor content being low. Also, the halo-containing resin compositions as shown in Table 4 are qualified for UL94 V-0 Flammability Test and improve other properties of substrate with bromine content being low. Therefore, the present resin compositions added with NPBX thermosetting resin in the present invention is of low cost. Further, lower curing temperature, higher resin crosslinking density and improvement on substrate properties are achieved by adding novolac resin curing agent.

## Claims

1. An epoxy resin varnish composition with high glass transition temperature for laminate plate, which is admixed with dihydrobenzoxazine thermosetting resin, comprising: (A) 20-70 wt% of dihydrobenzoxazine thermosetting resin, (B) 30-70 wt% of one or more epoxy resins , (C) 1-20 wt% of novolac resin curing agent, and (D) curing promoter, with usage being 0.1 PHR based on total amount of resin,
wherein the dihydrobenzoxazine thermosetting resin has the following formula: 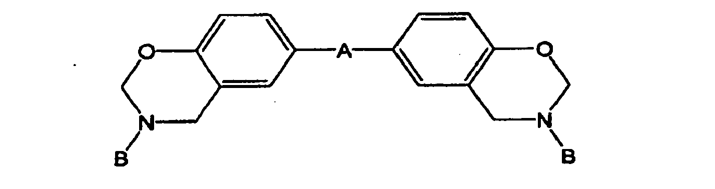 wherein A represents the following formula: 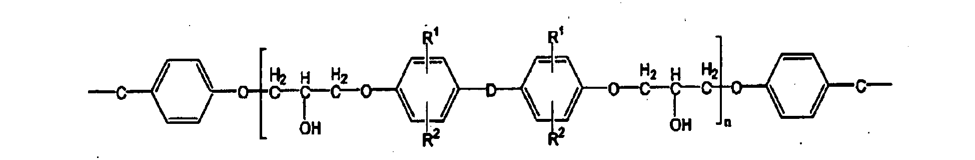 C represents 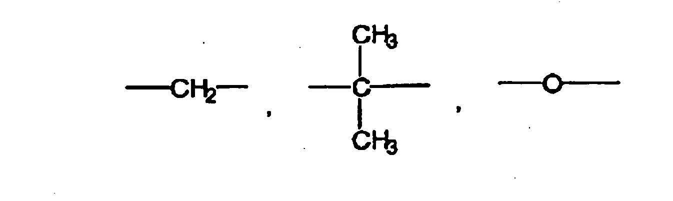 D represents 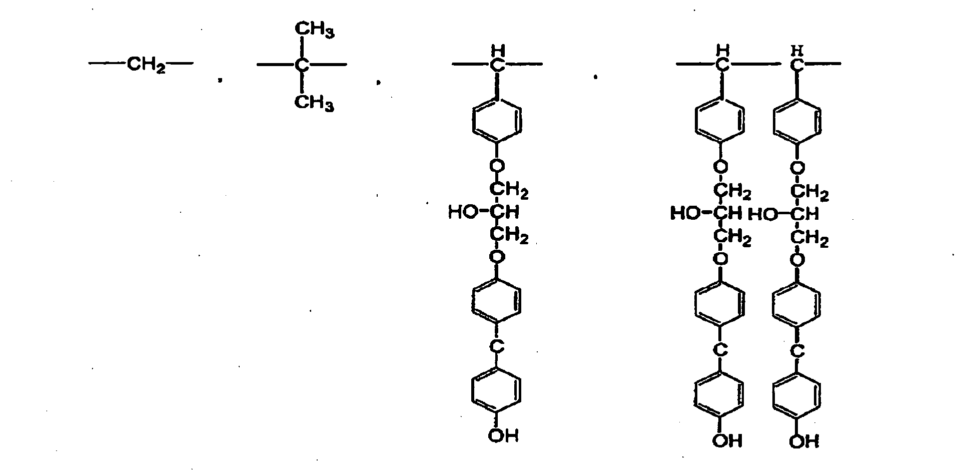 R¹ and R² each independently represents H, CH₃ or Br;
and B represents amines or diamines.

2. The varnish composition as claimed in Claim 1 , wherein (A) dihydrobenzoxazine thermosetting resin is obtained by reacting compounds (a) phenolic products obtained from the reaction of di-or multifunctional epoxy resin and di-functional phenolic compounds; (b) mono-or di-functional primary amines; (c) di-functional phenols; and (d) formaldehyde or paraformaldehyde.

3. The varnish composition as claimed in Claim 2, wherein (a) is phenolic product obtained from the reaction of di- or multifunctional epoxy resin and di-functional phenolic compounds with content of 5-40 wt% and of the following formula: 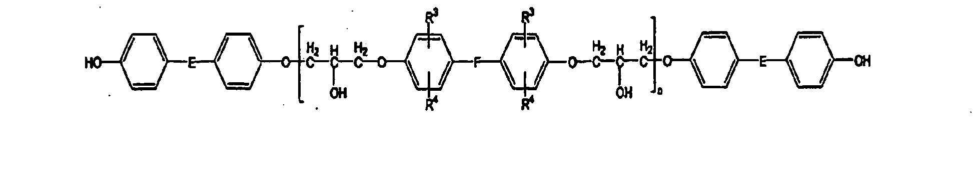 wherein F represents 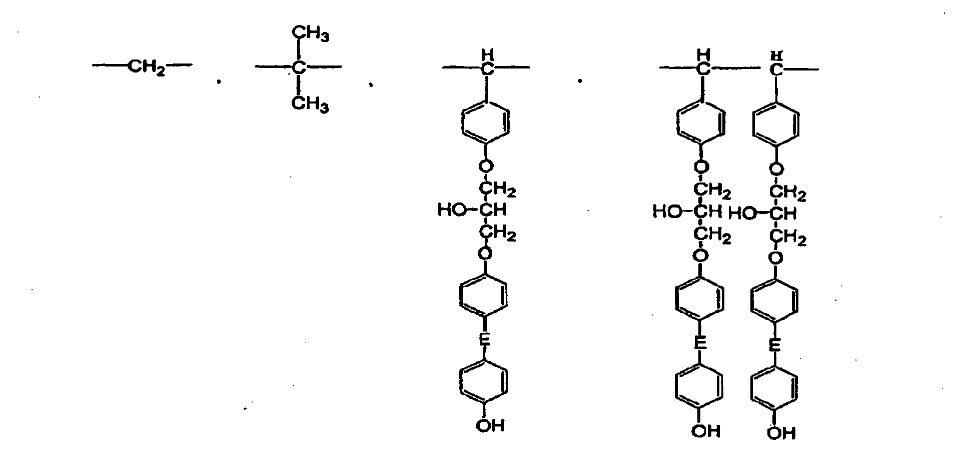 E represents 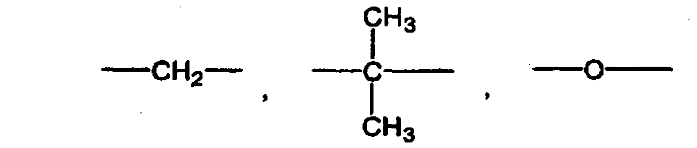 and R³ and R⁴ each independently represents H, CH₃ or Br.

4. The varnish composition as claimed in Claim 2, wherein (b) is a mono-or di-functional primary amine which content is 20-40 wt%.

5. The varnish composition as claimed in Claim 2, wherein (c) is a di-functional phenol with content of 15-70 wt%.

6. The varnish composition as claimed in Claim 2, wherein (d) is formaldehyde or paraformaldehyde with content of 10-30 wt%.

7. The varnish composition as claimed in Claim 2, wherein organic solvent is selected from alcohols, ketones, ethers, or hydrocarbons, preferably hydrocarbons such as toluene.

8. The varnish composition as claimed in Claim 1, wherein (B) is one or more epoxy resins with phosphor-containing or bromine-containing epoxy resins.

9. The varnish composition as claimed in Claim 8, wherein phosphor- containing epoxy resin is obtained by reacting phosphorus compounds and o-cresol formaldehyde novolac epoxy, in which epoxy equivalent is 350-480 g/eq with phosphor content of 2-5 wt%, at 30-70 wt% of the composition.

10. The varnish composition as claimed in Claim 8, wherein phosphor-containing epoxy resin is obtained by reacting phosphorus compounds and phenol novolac epoxy, in which epoxy equivalent is 340-460 g/eq with phosphor content of 2-5 wt%, at 30-70 wt% of the composition.

11. The varnish composition as claimed in claim 9 or 10, wherein the phosphorus compound is 9,10-dihydro-9-oxa-10-phosphorphenanthrene-10-oxo compound or DOPO.

12. The varnish composition as claimed in Claim 8, wherein bromine-containing epoxy resin is obtained by reacting tetrabromobisphenol A and liquid epoxy resin, in which bromine content is 14-25 wt%, at 30-70% of the composition.

13. The varnish composition as claimed in Claim 1, wherein (C) novolac resin curing agent is selected from phenol novolac resin , melamine phenol novolac , BPA phenol novolac , and tetraphenol ethane resin , preferably tetraphenol ethane resin, at 5-30 wt% of the composition.

14. The varnish composition as claimed in Claim 1, wherein ( D) curing promoter is imidazole and benzyldimethyl amine, preferably 2-methyl imidazole and 2-phenyl imidazole or 2-ethyl-4-methyl imidazole , at 0.01-0.15 PHR based on total amount of resin.

15. The varnish composition as claimed in Claim 1, wherein additive can be selected from inorganic fillers such as AI (OH)₃, SiO₂ or clay, titanium dioxide, barium titanate, strontium titanate, etc., at additional 30-50 wt%.

16. The varnish composition as claimed in Claim 1, wherein solvent for adjusting viscosity is selected from acetone, methyl ethyl ketone, 1-methoxy-2-propanol , isopropanol , or N, N-dimethylformamide.

17. The varnish composition as claimed in Claim 1, wherein said composition can be used for multilayer laminate plates, composite materials, printed circuit boards, inks, or packaging materials.

## Patentansprüche

1. Eine Epoxidharzlackzusammensetzung mit hoher Glasübergangstemperatur für eine Laminatplatte bzw. Verbundplatte, welche mit einem wärmeaushärtenden Harz aus Dihydrobenzoxazin vermischt ist, umfassend: (A) 20-70 Gew.-% eines wärmeaushärtenden Harzes aus Dihydrobenzoxazin, (B) 30 - 70 Gew.-% eines oder mehrerer Epoxidharze, (C) 1 - 20 Gew.-% eines Novolackharz-Härtungsmittels, und (D) einem Aushärtungsförderungsmittel, wobei sein Einsatz 0,1 PHR (pro hundert Anteile Harz), basierend auf der Gesamtmenge des Harzes ist,
wobei das wärmeaushärtende Harz aus Dihydrobenzoxazin die folgende Formel hat: 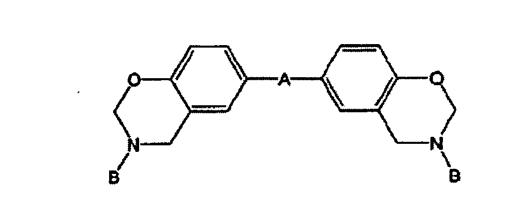 wobei A die folgende Formel darstellt: 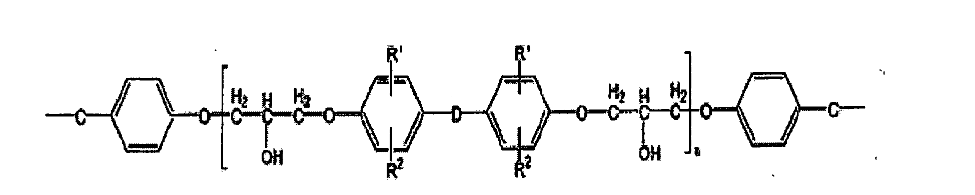 C Folgendes darstellt: 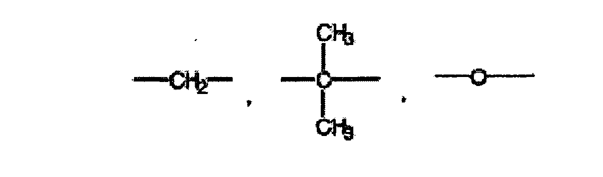 D das Folgende darstellt: 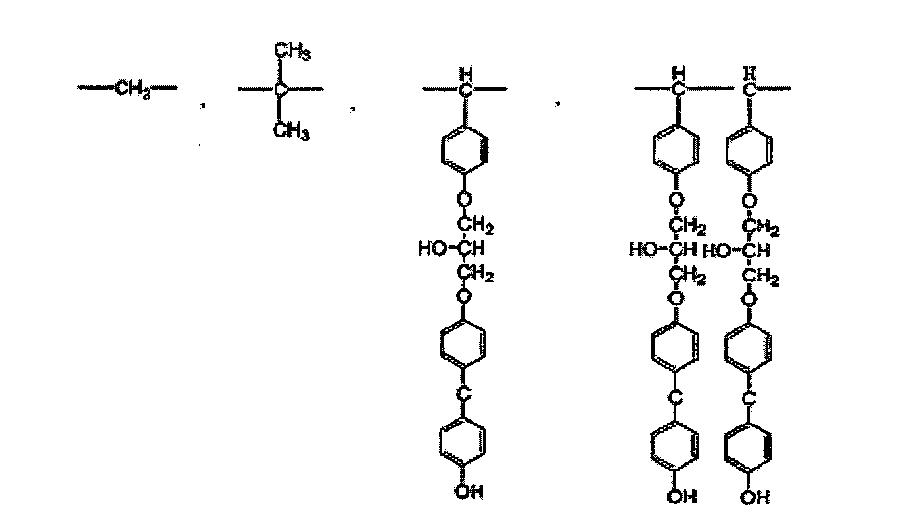 R¹ und R² jeweils unabhängigerweise H, CH₃ oder Br darstellen; und B Amine oder Diamine darstellt.

2. Die Lackzusammensetzung nach Anspruch 1, wobei das wärmeaushärtende Harz aus Dihydrobenzoxazin(A) durch Reaktion der Verbindungen (a) phenolische Produkte, erhalten aus der Reaktion aus di- oder multifunktionellem Epoxidharz und difunktionellen phenolischen Verbindungen; (b) mono- oder difunktionelle primäre Amine; (c) difunktionelle Phenole; und (d) Formaldehyd oder Paraformaldehyd erhalten wird.

3. Die Lackzusammensetzung nach Anspruch 2, wobei (a) ein phenolisches Produkt ist, erhalten aus der Reaktion aus di- oder multifunktionellem Epoxidharz und difunktionellen phenolischen Verbindungen mit einem Gehalt von 5 - 40 Gew.-% und mit der folgenden Formel: 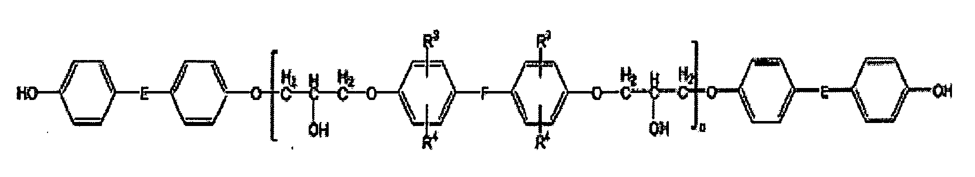 wobei F Folgendes darstellt: 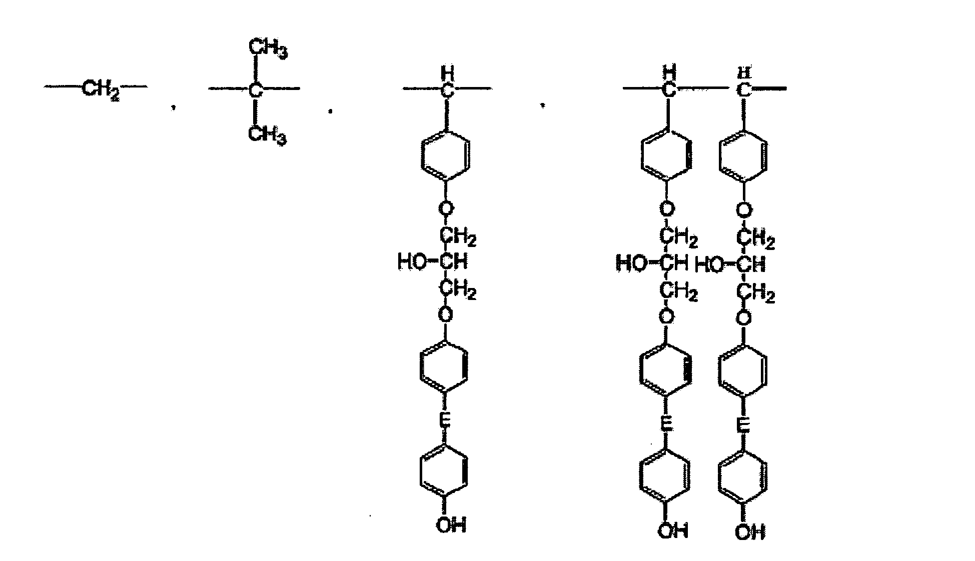 E Folgendes darstellt: 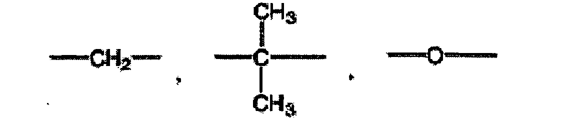 und R³ und R⁴ jeweils unabhängigerweise H, CH₃ oder Br darstellen.

4. Die Lackzusammensetzung nach Anspruch 2, wobei (b) ein mono- oder difunktionelles primäres Amin ist, wobei sein Gehalt bei 20 - 40 Gew.-% liegt.

5. Die Lackzusammensetzung nach Anspruch 2, wobei (c) ein difunktionelles Phenol mit einem Gehalt von 15 - 70 Gew.-% ist.

6. Die Lackzusammensetzung nach Anspruch 2, wobei (d) ein Formaldehyd oder Paraformaldehyd mit einem Gehalt von 10 - 30 Gew.-% ist.

7. Die Lackzusammensetzung nach Anspruch 2, wobei das organische Lösungsmittel ausgewählt ist aus Alkoholen, Ketonen, Ethern oder Kohlenwasserstoffen, bevorzugt Kohlenwasserstoffen wie etwa Toluol.

8. Die Lackzusammensetzung nach Anspruch 1, wobei (B) ein oder mehrere Epoxidharze mit phosphorhaltigen oder bromhaltigen Epoxidharzen ist.

9. Die Lackzusammensetzung nach Anspruch 8, wobei das phosphorhaltige Epoxidharz durch eine Reaktion von Phosphorverbindungen mit o-Cresol/Formaldehyd/Novolack-Epoxid erhalten worden ist und in welchem das Epoxidäquivalent bei 350 - 480 g/äq. mit einem Phosphorgehalt von 2 - 5 Gew.-% liegt, bei 30 - 70 Gew.-% der Zusammensetzung.

10. Die Lackzusammensetzung nach Anspruch 8, wobei das phosphorhaltige Epoxidharz durch Reaktion von Phosphorverbindungen und Phenolnovolackepoxid erhalten worden ist und in welchem das Epoxidäquivalent bei 340 - 460 g/äq. mit einem Phosphorgehalt von 2 - 5 Gew.-% liegt, bei 30 - 70 Gew.-% der Zusammensetzung.

11. Die Lackzusammensetzung nach Anspruch 9 oder 10, wobei die Phosphorverbindung eine 9,10-Dihydroxo-9-oxa-10-phosphorphenantren-10-oxo-Verbindung oder DOPO ist.

12. Die Lackzusammensetzung nach Anspruch 8, wobei das bromhaltige Epoxidharz durch Reaktion von Tetrabrombisphenol A und flüssigem Epoxidharz erhalten worden ist und in welchem der Bromgehalt bei 14 - 25 Gew.-% liegt, bei 30 - 70 Gew.-% der Zusammensetzung.

13. Die Lackzusammensetzung nach Anspruch 1, wobei das Novolackharzaushärtungsmittel (C) ausgewählt ist aus Phenolnovolackharz, Melaminphenolnovolack, BPA-Phenolnovolack und Tetraphenolethanharz, bevorzugt aus Tetraphenolethanharz, bei 5 - 30 Gew.-% der Zusammensetzung.

14. Die Lackzusammensetzung nach Anspruch 1, wobei das Aushärtungsförderungsmittel (D) Imidazol und Benzyldimethylamin, bevorzugt 2-Methylimidazol und 2-Phenylimidazol oder 2-Ethyl-4-methylimidazol bei 0,01-0,15 PHR (pro hundert Anteile Harz), basierend auf der Gesamtmenge des Harzes ist.

15. Die Lackzusammensetzung nach Anspruch 1, wobei das Additiv ausgewählt sein kann aus anorganischen Füllmitteln wie etwa Al(OH)₃, SiO₂ oder Lehm, Titandioxid, Bariumtitanat, Strontiumtitanat, usw., bei zusätzlichen 30 - 50 Gew.-%.

16. Die Lackzusammensetzung nach Anspruch 1, wobei das Lösungsmittel zur Einstellung der Viskosität ausgewählt ist aus Azeton, Methylethylketon, 1-Methoxy-2-propanol, Isopropanol oder N,N-Dimethylformamid.

17. Die Lackzusammensetzung nach Anspruch 1, wobei die Zusammensetzung für mehrschichtige Laminatplatten bzw. Verbundplatten, Kompositmaterialien, gedruckten Leiterplatten, Tinten oder Verpackungsmaterialien eingesetzt werden kann.

## Revendications

1. Composition de vernis à base de résine époxy ayant une température de transition vitreuse élevée pour une plaque stratifiée, qui est mélangée avec une résine thermodurcissable de dihydrobenzoxazine, comprenant : (A) de 20 à 70 % en poids de résine thermodurcissable de dihydrobenzoxazine, (B) de 30 à 70 % en poids d'une ou plusieurs résines époxy, (C) de 1 à 20 % en poids d'un agent durcissant de type résine novolac, et (D) un agent améliorant le durcissement utilisé en une quantité de 0,1 phr par rapport à la quantité totale de résine,
dans laquelle la résine thermodurcissable de dihydrobenzoxazine a la formule suivante : 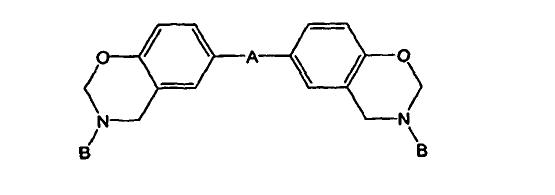 dans laquelle A représente la formule suivante : 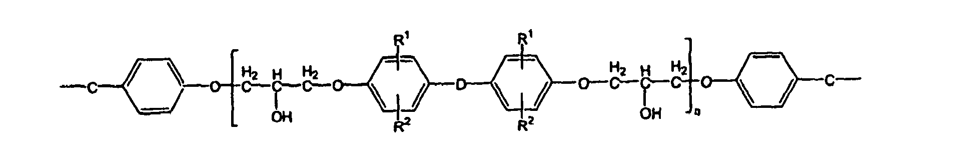 C représente 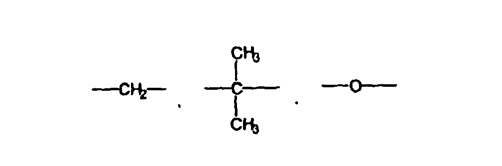 D représente 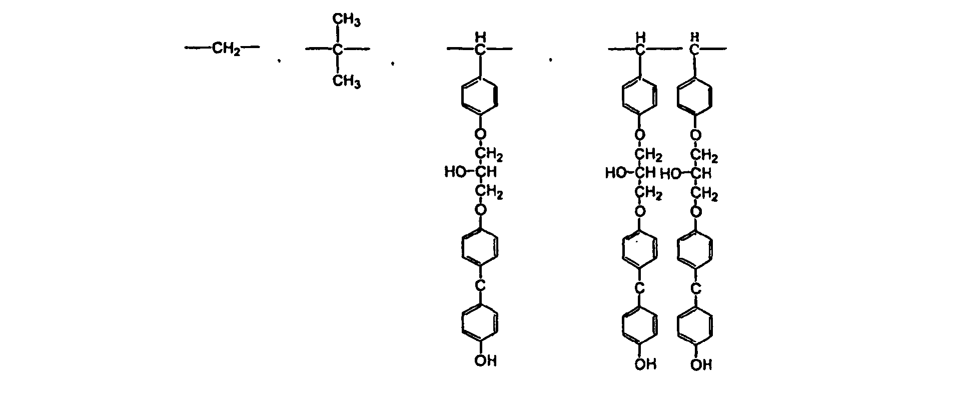 R¹ et R² représentent chacun indépendamment H, CH₃, ou Br ; et
B représente des amines ou des diamines.

2. Composition de vernis selon la revendication 1, dans laquelle (A) la résine thermodurcissable de dihydrobenzoxazine est obtenue en faisant réagir (a) des produits phénoliques, obtenus à partir de la réaction d'une résine époxy di- ou multifonctionnelle et de composés phénoliques difonctionnels ; (b) des amines primaires mono- ou difonctionnelles ; (c) des phénols difonctionnels ; et (d) du formaldéhyde ou du paraformaldéhyde.

3. Composition de vernis selon la revendication 2, dans laquelle (a) est un produit phénolique obtenu à partir de la réaction d'une résine époxy di- ou multifonctionnelle et de composés phénoliques difonctionnels avec une teneur de 5 à 40 % en poids et ayant la formule suivante : 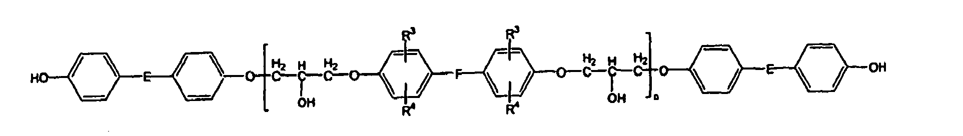 dans laquelle F représente 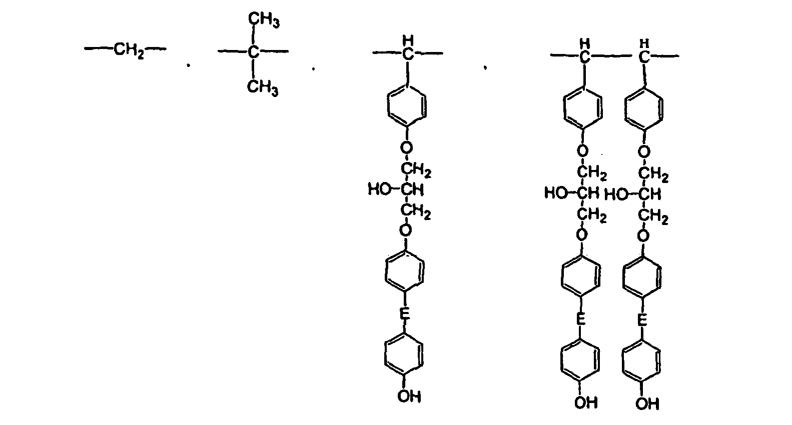 E représente 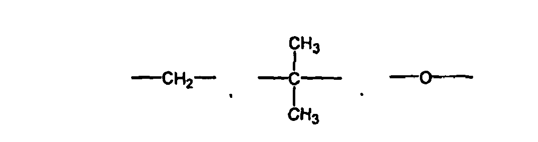 et R³ et R⁴ représentent chacun indépendamment H, CH₃ ou Br.

4. Composition de vernis selon la revendication 2, dans laquelle (b) est une amine primaire mono- ou difonctionnelle dont la teneur est de 20 à 40 % en poids.

5. Composition de vernis selon la revendication 2, dans laquelle (c) est un phénol difonctionnel avec une teneur de 15 à 70 % en poids.

6. Composition de vernis selon la revendication 2, dans laquelle (d) est le formaldéhyde ou le paraformaldéhyde avec une teneur de 10 à 30 % en poids.

7. Composition de vernis selon la revendication 2, dans laquelle le solvant organique est choisi parmi les alcools, les cétones, les éthers, ou les hydrocarbures, de préférence les hydrocarbures tels que le toluène.

8. Composition de vernis selon la revendication 1, dans laquelle (B) est une ou plusieurs résines époxy avec des résines époxy contenant du phosphore ou contenant du brome.

9. Composition de vernis selon la revendication 8, dans laquelle la résine époxy contenant du phosphore est obtenue en faisant réagir des composés phosphoreux et un o-crésol formaldéhyde novolac époxy, où l'équivalent d'époxy est de 350 à 480 g/éq, avec une teneur en phosphore de 2 à 5 % en poids, à 30 à 70 % en poids de la composition.

10. Composition de vernis selon la revendication 8, dans laquelle la résine époxy contenant du phosphore est obtenue en faisant réagir des composés phosphoreux et un phénol novolac époxy, où l'équivalent d'époxy est de 340 à 460 g/éq, avec une teneur en phosphore de 2 à 5 % en poids, à 30 à 70 % en poids de la composition.

11. Composition de vernis selon la revendication 9 ou 10, dans laquelle le composé phosphoreux est le composé 9,10-dihydro-9-oxa-10-phosphorphénanthrène-10-oxo ou DOPO.

12. Composition de vernis selon la revendication 8, dans laquelle la résine époxy contenant du brome est obtenue en faisant réagir du tétrabromobisphénol A et une résine époxy liquide, où la teneur en brome est de 14 à 25 % en poids, à 30 à 70 % en poids de la composition.

13. Composition de vernis selon la revendication 1, dans laquelle (C) l'agent durcissant de type résine novolac est choisi parmi les résines phénol novolac, mélamine phénol novolac, BPA phénol novolac, et tétraphénol éthane, de préférence la résine tétraphénol éthane, à 5 à 30 % en poids de la composition.

14. Composition de vernis selon la revendication 1, dans laquelle (D) l'agent favorisant le durcissement est un imidazole et une benzyldiméthylamine, de préférence le 2-méthylimidazole et le 2-phénylimidazole ou le 2-éthyl-4-méthylimidazole, à 0,01 à 0,15 phr par rapport à la quantité totale de résine.

15. Composition de vernis selon la revendication 1, dans laquelle l'additif peut être choisi parmi les charges inorganiques telles que Al(OH)₃, SiO₂ ou l'argile, le dioxyde de titane, le titanate de baryum, le titanate de strontium, etc., à 30 à 50 % en poids additionnels.

16. Composition de vernis selon la revendication 1, dans laquelle le solvant pour ajuster la viscosité est choisi parmi l'acétone, la méthyléthylcétone, le 1-méthoxy-2-propanol, l'isopropanol, ou le N,N-diméthylformamide.

17. Composition de vernis selon la revendication 1, dans laquelle ladite composition peut être utilisée pour des plaques stratifiées multi-couches, des matériaux composites, des cartes de circuit imprimé, des encres, ou des matériaux d'emballage.
